# EUROPÄISCHE PATENTSCHRIFT

(11) **EP 2 854 938 B1**
(45) Veröffentlichungstag und Bekanntmachung des Hinweises auf die Patenterteilung: **07.08.2019**
(21) Anmeldenummer: 13730135.4
(22) Anmeldetag: 27.05.2013
(51) Int. Cl.: A61N 1/20, A61B 18/12, A61N 1/05, A61B 18/14

(54) **THERAPEUTISCH ANWENDBARE GLEICHSTROMABGABEVORRICHTUNG**
DC OUTPUT APPARATUS WHICH CAN BE USED FOR THERAPEUTIC PURPOSES
DISPOSITIF DE DISTRIBUTION DE COURANT CONTINU UTILISABLE À DES FINS THÉRAPEUTIQUES

(30) Priorität: 25.05.2012 DE 102012010262
(43) Veröffentlichungstag der Anmeldung: 08.04.2015
(73) Patentinhaber: Molsberger, Albrecht, 40489 Düsseldorf (DE)
(72) Erfinder: Molsberger, Albrecht, 40489 Düsseldorf (DE)
(74) Vertreter: König, Gregor Sebastian
(86) Internationale Anmeldenummer: PCT/EP2013/060893
(87) Internationale Veröffentlichungsnummer: WO 2013/175021

(56) Entgegenhaltungen:
- DE-A1- 3 719 353
- US-A- 3 842 841
- US-A- 4 913 148
- US-A- 5 439 440
- US-A1- 2001 046 653
- US-A1- 2004 010 290
- US-A1- 2004 167 458
- US-B1- 6 277 116

## Beschreibung

Die vorliegende Erfindung betrifft eine Vorrichtung zur Abgabe von Gleichstrom. Die erfindungsgemäße Gleichstromabgabevorrichtung ist zur therapeutischen oder kosmetischen Behandlung des menschlichen oder tierischen Körpers einsetzbar. Die vorliegende Erfindung richtet sich außerdem auf die Gleichstromabgabevorrichtung zur spezifischen Anwendung in bestimmten Verfahren zur therapeutischen Behandlung des menschlichen oder tierischen Körpers. Die erfindungsgemäße Gleichstromabgabevorrichtung eignet sich insbesondere zur Behandlung von Entzündungen und/oder Schmerzen. Schließlich betrifft die Erfindung auch ein Kit und ein Verfahren zum Herstellen der Gleichstromabgabevorrichtung.

Viele medizinisch oder kosmetisch relevante Beeinträchtigungen des menschlichen oder tierischen Körpers sind lokal bedingt. Eine medizinisch relevante Beeinträchtigung liegt bei Krankheiten oder Störungen der Funktion des Körpers vor. In diesen Fällen ist grundsätzlich eine therapeutische Behandlung indiziert. Im Fall von Reizzuständen, worunter im Rahmen der vorliegenden Erfindung relativ geringfügige Beeinträchtigungen oder Beschwerden des menschlichen oder tierischen Körpers verstanden werden, die keine Krankheit oder Funktionsstörung darstellen und die nicht therapiewürdig sind, ist oftmals zumindest eine kosmetische, nicht-therapeutische Behandlung sinnvoll.

Die vorliegende Erfindung betrifft sowohl die nicht-therapeutische kosmetische Behandlung von durch (vorwiegend lokale) Reizzustände verursachten kosmetischen Beeinträchtigungen als auch die Schaffung von neuen Therapiemöglichkeiten für (vorwiegend lokale) medizinisch relevante Beeinträchtigungen des menschlichen oder tierischen Körpers.

Der Begriff der Therapie umfasst hierbei auch die Prophylaxe. "Lokal" bedeutet, dass am oder im Körper ein bestimmtes Areal des Reizzustandes oder der medizinisch relevanten Beeinträchtigung feststellbar ist. Dieses Areal ist vorzugsweise lokal umschrieben und bevorzugt exakt identifizierbar. Das Areal (z.B. Reizareal, Entzündungsareal, Schmerzareal) geht mit bestimmten Symptomen einher und löst die Beeinträchtigung bzw. die Beschwerden aus oder ist damit assoziiert.

Beeinträchtigungen des menschlichen oder tierischen Körpers, die von lokalen Entzündungs- bzw. Schmerzzuständen herrühren, sind beispielsweise aseptische Entzündungen - oft bedingt durch lokale Überlastungen (Distorsionen, Ansatztendinosen) oder auch neuropathische Schmerzen. Im orthopädischen Bereich treten lokale Entzündungs- und Schmerzzustände insbesondere auf im Zusammenhang mit Gewebsverletzungen, insbesondere von Muskeln (z.B. Muskeltraumata), Nerven, Haut oder Halteapparat, Gefäßverletzungen oder auch im Zusammenhang mit Nervenentzündung, Entzündungen der Sehnen oder Knochen oder Narbenbildung. Oft besteht dabei ein lokal umschriebenes Areal, in dem die Beschwerden feststellbar sind.

Erfüllt eine Beeinträchtigung das Kriterium einer Erkrankung oder Störung der Funktion des Körpers, so ist es in der Regel angezeigt, sie therapeutisch zu behandeln. Beispiele für eine lediglich kosmetisch relevante Beeinträchtigung sind dagegen Faltenbildung, etwa durch erhöhten Muskeltonus bedingt, Fehl- und Schonhaltungen oder lediglich kosmetisch störende Hautveränderungen wie etwa Rötungen.

Verfahren und Mittel zur therapeutischen oder kosmetischen Behandlung von medizinisch oder kosmetisch relevanten Beeinträchtigungen und Beschwerden sind an sich bekannt. Auch wenn z.B. Physiotherapie in manchen Fällen Linderung verschaffen kann, werden hierfür doch in der Regel bestimmte pharmazeutische Wirkstoffe eingesetzt, in erster Linie Cortison, nichtsteroidale Antirheumatika, Analgetika und verwandte Stoffe. Diese haben neben der gewünschten (lokalen) Wirkung meist auch unerwünschte lokale Nebenwirkungen (bei lokalen Gaben von Cortison etwa aseptische Entzündungen) und/oder systemische Nebenwirkungen und können den Stoffwechsel und Hormonhaushalt nachteilig beeinflussen. In vielen Fällen können die herkömmlichen Therapieverfahren einen chronischen Krankheitsverlauf nicht abwenden. Es ist daher sinnvoll, Alternativen zum Einsatz (exogener) Wirkstoffe in Betracht zu ziehen.

Es sind auch Therapieoptionen bekannt, die verstärkten Gebrauch von den intrinsischen Heilungskapazitäten des menschlichen oder tierischen Körpers machen. Eine weit verbreitete Technik ist die auf die traditionelle chinesische Medizin (TCM) zurückgehende Akupunktur und deren Abwandlungen.

Akupunktur ist für die Behandlung bestimmter Beschwerden, etwa bei chronischem Schmerz (z.B. Kopfschmerz, Migräne) allgemein als effektiv und risikoarm anerkannt. So zahlen etwa alle deutschen gesetzlichen Krankenkassen seit dem 1. Januar 2007 insbesondere auf Grundlage der Ergebnisse großer prospektiver und randomisierter Studien (GERAC, German Acupuncture Trials) Akupunkturbehandlungen bei chronischen Kreuzschmerz und chronischem Knieschmerz bei Gonarthrose. Private Krankenversicherungen bezahlen Akupunkturleistungen zur Schmerzbehandlung und nach Einzelfallentscheidung meist auch für weitere Indikationen. Die "Cochrane-Reviews" von 2009 bezeichnen Akupunktur als "eine wertvolle nicht pharmakologische Therapiemöglichkeit bei Patienten mit häufigem episodischem Spannungskopfschmerz" und stellen fest, dass die "Akupunktur bei Migräne mindestens so wirksam, möglicherweise auch wirksamer, als eine medikamentöse prophylaktische Therapie ist, und dies bei geringeren unerwünschten Wirkungen".

Es zeigte sich in den GERAC-Studien, dass kein signifikanter Unterschied bestand zwischen einer Akupunktur an Punkten, die den Vorgaben der TCM folgt und einer Akupunktur an anderen Punkten (sogenannte "Scheinakupunktur"). Positive therapeutische oder kosmetische Effekte sind bei der Anwendung auf lokale Entzündungs- und Schmerzzustände für beide Formen der Akupunktur nachgewiesen. Heutzutage sind auch Akupunkturformen geläufig, die sich nicht am traditionellen theoretischen Hintergrund der TCM orientieren.

Endogene physiologische elektrische Felder sind in der Biologie bekannt. Solche Felder liegen im Bereich von 70 mV/mm (Nervenwachstum bei Hühnern), 140 mV/mm (Wundheilung bei Ratten), 600 mV/mm (Augenlinse von Wirbeltieren) bis 1500 mV/mm (Entwicklung des Neuronalrohrs beim Lurch Axolotl). Je nach Innenwiderstand des betreffenden biologischen Gewebes ergeben sich hierbei Ströme von 10-200 µA. Endogene elektrische Felder bauen sich für eine Zeit von Stunden bis Wochen beispielsweise im Wundbereich, im Bereich des aktiven Zellwachstums sowie bei der Zellmigration auf und scheinen für die Regulation des Zellverhaltens essenziell zu sein.

Der Einsatz von exogenen elektrischen Feldern in der Medizin und Kosmetik ist dem Grunde nach bekannt. Eingesetzt werden hierbei regelmäßig starke und/oder zeitlich veränderliche Felder, wobei die zeitliche Veränderlichkeit etwa durch Wechselspannung oder kurze Gleichspannungsimpulse bewirkt wird. Diese bislang therapeutisch eingesetzten starken elektrischen Felder werden beispielsweise durch hohe Spannungen und regelmäßig durch starke Ströme erzeugt. In diesem Zusammenhang werden Wechselstrom- und Impulsstromgeräte verwendet, um elektrolytischen Effekten an den eingesetzten Elektroden und insbesondere am Körpergewebe entgegenzuwirken.

Bekannt ist etwa die transkutane elektrische Nervenstimulation (TENS). Hierbei werden niederfrequente (1-100 Hz) biphasische Wechselstromimpulse zur Schmerzlinderung eingesetzt, primär zur kurzfristigen "Elektroanalgesie". Die Spannung beträgt bis zu 70 V bei etwa 250 µs Impulsdauer, die Stromstärke bis zu etwa 90 mA. Die Wirkung beruht in erster Linie auf einer Steigerung der zentralen Ausschüttung von Endorphinen. Es ist unklar, ob darüber hinaus auch lokale und längerfristige Effekte im betroffenen Gewebe erzielt werden.

Bekannt ist auch die Elektroakupunktur. Ihr Wirkmechanismus zielt auf die Ausschüttung zentraler schmerzlindernder Substanzen, insbesondere von Enkephalinen, Endorphinen und Dynorphinen.

Wie im Dokument US 2004/0111128 A1 beschrieben, verwendet auch die Elektroakupunktur Wechselströme. Bei der Elektroakupunktur wird ein niederfrequenter Reizstrom gesetzt (Springer Lexikon der Medizin), hierbei ist die Frequenz des elektrischen Signals fest oder variabel (2-10.000 Hz). Eingesetzt werden hierbei wie bei TENS verhältnismäßig starke Ströme, die zwischen 2 und 15 mA liegen. Diese können in dieser Intensität nur gepulst appliziert werden mit einer Impulsdauer von ca. 0,3 - 0,6 ms. Um bei diesen hohen Strömen elektrolytische Effekte am Übergang zwischen Elektrode und biologischem Gewebe zu vermeiden, wird die Polung gewechselt (Wechselstrom). Dementsprechend umfassen die elektrischen Parameter bei der Elektroakupunktur in jedem Fall Frequenz und Intensität (siehe eine unter der URL http://www.icmart.org/index.php?id=198,0,0,1,0,0 abrufbare und von einem Kongress des International Council on Medical Acupuncture and Related Techniques stammende Zusammenfassung über Parameter der Elektroakupunktur).

Zusammenfassend sind die bekannten Geräte zur TENS oder Elektroakupunktur schaltungstechnisch aufwendig, sie arbeiten mit hohen Strömen, kurzen Impulsen und Wechselstrom bestimmter Frequenzen. Ungeachtet dessen ist die Stimulationsdosis ist oft nicht kontrollierbar. Diese Mittel und Verfahren des Stands der Technik zielen auf eine Schmerzlinderung, die auf einer zentralen analgetischen Wirkung beruht. Auf eine lokale Wirkung (etwa eine entzündungshemmende oder regenerationsfördernde) stellen sie nicht ab.

In der Tumortherapie ist eine Gleichstrom-Galvanotherapie mit hohen Stromstärken von 60-80 mA bei einer Spannung von 6-35 V bekannt. Bei dieser Therapie soll es zu einer Zerstörung des Tumorgewebes kommen, beispielsweise durch Nekrotisierung. Eine Zerstörung von Gewebe ist hier also kein zu vermeidender unerwünschter Effekt, sondern im Gegenteil ausdrücklich erwünscht. Das Verfahren macht sich die erhöhte Leitfähigkeit von Tumorgewebe im Vergleich zu gesundem Gewebe zu Nutze, so dass sich der Stromfluss selektiv im Tumorgewebe konzentrieren und dort den Zerfall des Tumors durch elektrolytische und nekrotisierende Effekte bewirken soll.

Gleichstrom wird auch zum transkutanen Transport ionisierbarer Medikamente (Iontophorese) eingesetzt. Zum Einsatz kommen Spannungen von etwa 36-60 V und Ströme von etwa 10-30 mA. Um hier lokale Gewebsschädigungen zu vermeiden und eine hohe Dosis an Wirkstoffen elektrophoretisch transportieren zu können, werden großflächige Hautelektroden auf die Haut gesetzt.

Ferner ist eine Anwendung von breitflächigen feuchten Zellstoffelektroden auf der Kopfhaut zur Stimulation des Zentralnervensystems bekannt, zum Beispiel bei Tinnitus (transkranielle Gleichstromstimulation, tDCS). Hierbei wird eine Stromstärke bis 1 mA und eine Spannung von 8-25 V bei konstantem und pulsierendem Strom eingesetzt.

Es ist bekannt, dass die entsprechenden durch schwachen Gleichstrom erzeugten elektrischen Felder das Gefäßwachstum fördern, unter anderem über die Ausschüttung von VEGF und ihren Einfluss auf Endothelzellen. Sie führen zu einer Bewegung und Neuanordnung von Zellmembranrezeptoren, erhöhen die Teilungsrate bestimmter Zellen und beschleunigen die Zellwanderung von Epithelzellen. Diese Zellwanderung erfolgt von der Anode (vom positiven Pol) weg und zur Kathode (zum negativen Pol) hin. Im Tierexperiment gibt es Hinweise, dass die periphere Nervenregeneration nach Rückenmarkstrauma beschleunigt werden kann, wobei die Axone der Nervenzellen zur Kathode hin wachsen, die über etwa drei Wochen kopfwärts liegen muss. Klinische Studien am Menschen weisen auf eine Beschleunigung der Wundheilung durch elektrische Felder hin.

US 3842841 A offenbart ein Gerät zur Knochenheilung, das die Merkmale des Oberbegriffs von Anspruch 1 offenbart, aber nur eine Nadel als erste Elektrode verwendet.

Der vorliegenden Erfindung liegt das technische Problem zu Grunde, neuartige Mittel und Verfahren bereitzustellen, mit denen lokale Beeinträchtigungen des menschlichen oder tierischen Körpers, insbesondere solche, die durch Entzündungen und/oder Schmerzen verursacht werden, gelindert oder beseitigt werden können.

Vorzugsweise erlauben die erfindungsgemäßen Mittel und Verfahren eine effektivere, sicherere, reproduzierbarere und/oder nebenwirkungsärmere Anwendung und/oder haben eine längerfristige Wirkung als die Mittel und Verfahren des Stands der Technik.

Das technische Problem wird gemäß einem ersten Aspekt der vorliegenden Erfindung gelöst durch eine Gleichstromabgabevorrichtung wie in Anspruch 1 definiert. Weitere Ausführungsbeispiele sind in den abhängigen Ansprüchen definiert.

Der Begriff "Batterie" umfasst im Rahmen der vorliegenden Erfindung neben Batterien mit einer Spannung von vorzugsweise 1,2 V (etwa Nickel-Metallhydrid-Batterien) bis 1,5 V (etwa Alkali-Mangan- oder Zink-Kohle-Batterien), alleine oder in bevorzugt zwei-, drei- oder vierfacher Ausführung hintereinander geschaltet) auch Akkumulatoren und galvanische Zellen.

Unter einer "Klebeelektrode" wird vorliegend eine flächige Elektrode verstanden, die auf die (gegebenenfalls enthaarte) Hautoberfläche aufbringbar und dort fixierbar ist (gegebenenfalls unter Verwendung eines die Leitfähigkeit vermittelnden oder verbessernden Zusatzmaterials wie Elektrodengel oder Elektrodenpaste), vorzugsweise durch Aufkleben.

Eine flächige Elektrode wird wird für die Zwecke der vorliegenden Anmeldung zuweilen auch als "Pad" bezeichnet.

Auch offenbart wird die erfindungsgemäße Gleichstromabgabevorrichtung zur Anwendung bei der Behandlung von Entzündungen und/oder Schmerzen, insbesondere der Muskeln, Nerven, Sehnen oder Knochen.

Anders ausgedrückt offenbart wird beispielhaft auch die Verwendung der erfindungsgemäßen Gleichstromabgabevorrichtung zur Behandlung von Entzündungen und/oder Schmerzen, insbesondere der Muskeln, Nerven, Sehnen oder Knochen bzw. die Verwendung der erfindungsgemäßen Gleichstromabgabevorrichtung zur Herstellung einer therapeutischen Vorrichtung zur Behandlung von Entzündungen und/oder Schmerzen, insbesondere der Muskeln, Nerven, Sehnen oder Knochen. Dieser Aspekt der vorliegenden Erfindung betrifft auch ein Verfahren zur Behandlung von Entzündungen und/oder Schmerzen, insbesondere der Muskeln, Nerven, Sehnen oder Knochen eines Patienten, umfassend das Einwirkenlassen der erfindungsgemäßen Gleichstromabgabevorrichtung auf den Körper des Patienten.

Das der vorliegenden Erfindung zu Grunde liegende technische Problem wird gemäß einem zweiten Aspekt auch gelöst durch ein Kit zum Herstellen einer erfindungsgemäßen Gleichstromabgabevorrichtung wie in Anspruch 10 definiert.

Der Begriff "umfassend" beinhaltet auch die Bedeutung "bestehend aus" und hat in bevorzugten Ausführungsformen letztere Bedeutung, außer, wo sich aus dem Kontext zwingend etwas anderes ergibt. Entsprechendes gilt für Begriffsvarianten wie etwa "umfassen" und "bestehen aus".

Unter Gleichstrom wird vorliegend ein elektrischer Strom verstanden, dessen Richtung sich nicht ändert und dessen zeitgemittelte Stromstärke sich unter gleichbleibenden Rahmenbedingungen im Wesentlichen nicht ändert. Vorzugsweise ist der Gleichstrom ein "reiner" Gleichstrom, dessen Stromstärke sich unter gleichbleibenden Rahmenbedingungen im Wesentlichen oder überhaupt nicht ändert. Es sind allerdings auch gewisse zeitliche Schwankungen möglich, insbesondere ein "pulsierender" Gleichstrom, bei dem die Stromstärke periodisch um einen bestimmten Mittelwert pendelt, ohne dass sich dabei allerdings die Stromrichtung ändert. Der Gleichstrom ist also vorzugsweise ein um einen zuvor (vorzugsweise konstant) eingestellten Wert undulierender Gleichstrom, Die Undulation erfolgt vorzugsweise mit einer Frequenz zwischen 0,001 und 10 Hz, insbesondere zwischen 0,01 und 1 Hz, beispielsweise 0,1 Hz. Vorzugsweise ist sie rechteck-, sägezahn- und insbesondere sinusförmig. Vorzugsweise beträgt dabei die Auslenkung des Gleichstroms 50% des zuvor eingestellten Werts (d.h. die Werte bewegen sich zwischen 150% und 50% des zuvor eingestellten Werts), insbesondere 40%, 30%, 20%, 15%, 10%, 7,5%, 5%, 2,5% oder 1%.

Die vorliegende Erfindung beruht auf der Entdeckung, dass schwacher Gleichstrom die erwähnten medizinisch oder kosmetisch relevanten Beeinträchtigungen und Beschwerden bessern kann, wenn er über eine Elektrode in einem lokalen elektrischen Gleichspannungsfeld auf den Körper wirkt. Die Wirkungen treten bereits ein, wenn der Gleichstrom sehr schwach ist. Eine besonders gut reproduzierbare Wirkung ist erzielbar, wenn der Gleichstrom konstant ist. Je nach Anwendung erzielen nadelförmige oder auch flächige Elektroden besonders gute Effekte (z.B. zwei nadelförmige Elektroden, zwei flächige Elektroden oder auch eine nadelförmige in Kombination mit einer flächigen Elektrode, wobei eine nadelförmige Elektrode optional eine Mehrzahl von Nadeln umfasst und/oder eine flächige Elektrode optional eine Mehrzahl von flächigen Gebilden umfasst). Das erfindungsgemäße angewandte elektrische Feld liegt in der Größenordnung endogener und physiologischer elektrischer Felder.

Vorteilhaft ist der Einsatz von einer oder mehreren Nadeln als Elektrode. Ferner ist ein Konstanthalten der Stromstärke vorteilhaft. Die Stromstärke bestimmt die Stärke des elektrischen Felds im Gewebe. Bei konstanter Stromstärke (im Gegensatz zu beispielsweise der Einstellung einer konstanten Spannung) können etwaige Schwankungen des Widerstands keine Schwankungen der Stromstärke und insbesondere keine Stromspitzen hervorrufen. Auch interindividuelle Unterschiede des Widerstands können (im Gegensatz zur Einstellung einer konstanten Spannung) nicht zu unterschiedlichen Stromstärken führen. Es wurde gefunden, dass bei einer nicht konstanten Stromstärke die erzielten Behandlungsergebnisse von Fall zu Fall in gewissem Umfang schwanken, ohne allerdings den grundsätzlichen Behandlungserfolg in Frage zu stellen.

Vorzugsweise umfasst die erfindungsgemäße Gleichstromabgabevorrichtung (wie oben beschrieben) ein Mittel zum Konstanthalten der Stromstärke bei der Abgabe des Gleichstroms (insbesondere im Fall der Veränderung eines zwischen den Elektroden anliegenden Widerstands). Dieses Mittel ist dazu ausgebildet, bei der Abgabe des Gleichstroms die Stromstärke insbesondere dann konstant zu halten, wenn sich der zwischen den Elektroden anliegende Widerstand ändert. Ohne ein solches Mittel besteht häufig die Situation, dass sich der elektrische Widerstand des Körpergewebes (etwa der Haut) während der Behandlung ändert und die Stromstärke dann schwankt. Es ist eine Erkenntnis der vorliegenden Erfindung, dass unter Verwendung eines Mittels zum Konstanthalten der Stromstärke das Ergebnis besonders gut reproduzierbar ist. Durch ein solches Mittel wird auch bei einem sich ändernden Widerstand ein konstanter Stromfluss gewährleistet und dadurch ein gleichbleibender Behandlungserfolg erzielt. Außerdem wurde gefunden, dass ohne ein Mittel zum Konstanthalten der Stromstärke aufgrund eines individuell verschiedenen Widerstands zwischen Haut und Gewebe, der meist im Bereich von 3-40 kΩ liegt, bei verschiedenen Individuen unterschiedliche Stromstärken beobachtbar sind und dadurch die Behandlungserfolge in gewissem Rahmen variieren. Unter Verwendung des Mittels zum Konstanthalten der Stromstärke wird der gleichbleibende Behandlungserfolg unabhängig vom individuell verschiedenen Widerstand zwischen Haut und Gewebe erzielt.

Durch die erfindungsgemäße Gleichstromabgabevorrichtung wird eine Besserung oder Beseitigung von kosmetisch oder medizinisch relevanten Beeinträchtigungen und Beschwerden des Körpers ermöglicht. Diese sind vorzugsweise Entzündungen und/oder Schmerzen und insbesondere lokal bedingt. Erfindungsgemäß kann die Verwendung exogener pharmazeutischer Wirkstoffe bzw. Medikamente reduziert oder vollständig vermieden werden. Dadurch treten die unerwünschten Nebenwirkungen solcher Stoffe in verringerten Maß oder gar nicht auf. Die Beeinträchtigungen und Beschwerden werden bei Einsatz der erfindungsgemäßen Gleichstromabgabevorrichtung dauerhaft oder zumindest langfristig gelindert oder beseitigt oder ihnen vorgebeugt. Bei Wiederholung der Anwendung kann die Wirkung oftmals bis zur dauerhaften Freiheit von den Beeinträchtigungen gesteigert werden.

Der Einsatz der erfindungsgemäßen Gleichstromabgabevorrichtung ist risikoarm, effektiv und arm an oder frei von Nebenwirkungen. Die Wirkung tritt schnell und voraussagbar ein. Die abgegebene Stromdosis ist exakt kontrollierbar. Vorteilhaft ist zudem, dass erfindungsgemäß auch eine Regeneration von durch chronische Entzündungen oder degenerative Prozesse geschädigtem Gewebe erlaubt wird. Die Wirksamkeit gegen Entzündungen und Schmerzen ist erfindungsgemäß erheblich besser als bei der Elektroakupunktur entsprechend dem Stand der Technik.

Die erfindungsgemäße Gleichstromabgabevorrichtung hat bei Anwendung am menschlichen oder tierischen Körper insbesondere eine antiphlogistische und analgetische Wirkung, die beispielsweise bei der Behandlung von (insbesondere lokalen) Entzündungen und Schmerzen, vor allem der Muskeln, Nerven, Sehnen oder Knochen vorteilhaft ist. Behandelbar sind etwa aseptische Entzündungen, Nervenschmerzen (z.B. neuropathischer Schmerz), Kopfschmerz und orthopädische Indikationen, wie etwa Schmerzen an der Brustwirbelsäule oder Schulter, Rückenschmerzen oder Sehnenschmerzen (etwa Tennisarm). Die Entzündungen/Schmerzen können beispielsweise im Zusammenhang stehen mit Gewebsverletzungen (z.B. Muskeln, Nerven, Haut oder Halteapparat, Gefäße), Nervenentzündung, Entzündungen der Sehnen oder Knochen und Narbenbildung.

Die erfindungsgemäße Gleichstromabgabevorrichtung erlaubt ein Behandlungskonzept, das auf der Akupunktur aufbaut. Es kann separat angewendet oder in den üblichen Kontext einer Akupunkturbehandlung eingebunden werden. Ein solches Konzept kann die Akupunktur fortentwickeln und so die entsprechende therapeutische oder kosmetische Behandlung von Beschwerden bzw. Beeinträchtigungen verbessern.

Erfindungsgemäß können mit der erfindungsgemäßen Gleichstromabgabevorrichtung menschliche oder tierische Körper von Patienten. Der Begriff "Patient" ist nicht einschränkend auf eine therapeutische Behandlung zu verstehen, sondern deckt auch eine kosmetische Behandlung ab. Bevorzugte Patienten sind Säugetiere wie Pferde, Hunde, Katzen oder Kamele und insbesondere Menschen.

Bei einem typischen Behandlungsablauf wird zunächst der schmerzende/entzündete Bereich lokalisiert. Beispielsweise werden eine oder mehrere (Metall-)Nadeln dort eingestochen und gegebenenfalls elektrisch miteinander verbunden. Die Spitze(n) der Nadel(n) kann/können auf oder außerhalb von Akupunkturpunkten liegen. Die Nadel(n) wird/werden als erste Elektrode mit einem Pol, vorzugsweise dem negativen Pol der Gleichstromquelle verbunden. Der andere Pol wird mit der zweiten Elektrode verbunden, die vorzugsweise eine Oberflächen-Klebeelektrode in einem anderen Bereich des Körpers ist. Eine solche Oberflächenelektrode ("Pad") wird vorzugsweise über große Muskelgruppen oder Fettschichten gelegt, damit nicht einzelne Nerven durch die Oberflächenelektrode gereizt werden. Ein vorzugsweise konstanter Strom wird zur Behandlung angelegt. Typischerweise lässt der Schmerz bzw. die Entzündung etwa 2 h nach der Behandlung nach.

Das Konstanthalten der Stromstärke ist vorzugsweise ein automatisiertes Konstanthalten. Die erfindungsgemäße Gleichstromabgabevorrichtung enthält demnach vorzugsweise ein automatisiertes Mittel zum Konstanthalten der Stromstärke bei der Abgabe des Gleichstroms (insbesondere im Fall der Veränderung eines zwischen den Elektroden anliegenden Widerstands).

Der elektrische Widerstand R bei der Behandlung wird in erster Linie vom Kontakt der Elektroden mit der Haut und gegebenenfalls vom direkten Umfeld der Nadel(n) bestimmt. Häufig verändert sich der Widerstand im Laufe der Behandlung. Um dennoch eine gleichbleibende Stromstärke I zu gewährleisten, bestehen unter anderem die Möglichkeiten, die Kontaktfläche zwischen Elektroden und Körpergewebe zu verändern, beispielsweise durch Verändern des Anpressdrucks der zweiten Elektrode, oder einen internen Widerstand der erfindungsgemäßen Gleichstromabgabevorrichtung zu verändern.

Vorzugsweise allerdings wird eine Konstanz der Stromstärke I durch entsprechende Veränderung der Spannung U gewährleistet.

Ein bevorzugtes Mittel zum Konstanthalten der Stromstärke in der erfindungsgemäßen Gleichstromabgabevorrichtung ist automatisiert und als Regler ausgestaltet, der beispielsweise aus analogen Bauteilen oder als integrierter Schaltkreis aufgebaut sein kann. Ein solcher Regler umfasst vorzugsweise ein Mittel zum Messen der Ist-Stromstärke (beispielsweise in der Zuleitung zur ersten Elektrode), ein Mittel zum Bestimmen einer Abweichung von einer vorbestimmten Soll-Stromstärke und ein Mittel zum Einstellen einer Korrektur der Spannung U entsprechend der Abweichung, insbesondere proportional zur Abweichung (Proportionalregler).

Erfindungsgemäß bevorzugte Gleichstromquellen sind zum Beispiel Batterien (wie oben definiert). Eine bevorzugte Batterie hat eine Spannung von 1,2 bis 1,5 V und liegt alleine oder in bevorzugt zwei-, drei- oder vierfacher Ausführung hintereinander geschaltet vor. Vorzugsweise werden Batterien in Abwesenheit eines Mittels zum Konstanthalten der Stromstärke bei der Abgabe des Gleichstroms eingesetzt, um eine besonders einfache und dennoch bereits wirksame Gleichstromabgabevorrichtung herzustellen. Batterien können alternativ jedoch auch in Verbindung mit einem Mittel zum Konstanthalten der Stromstärke bei der Abgabe des Gleichstroms eingesetzt werden. Weitere erfindungsgemäß bevorzugte Gleichstromquellen sind Netzteile oder Konstantstromquellen. Eine besonders bevorzugte Gleichstromquelle ist beispielsweise in dem von der neuroConn GmbH (Ilmenau, Deutschland) unter der Bezeichnung "DC-Stimulator" vertriebenen Gerät enthalten. Dieses Gerät wird im Stand der Technik zur transkraniellen Gleichstromstimulation (tDCS) des Gehirns angewendet. Es umfasst ein automatisiertes Mittel zum Konstanthalten der Stromstärke und wird in einem Kit zusammen mit zwei Schwammelektroden zum Auflegen auf den Kopf zur transkraniellen Gleichstromstimulation angeboten, nicht aber zur Elektroakupunktur. Die enthaltene Stromquelle ist unterschiedlich zu den im Stand der Technik für die Elektroakupunktur eingesetzten Stromquellen, da sie Gleichstrom statt Wechselstrom liefert und die abgegebene Stromstärke außerdem wesentlich geringer ist.

Vorzugsweise umfasst die erfindungsgemäße Gleichstromabgabevorrichtung einen veränderbaren inneren Widerstand. zum Einstellen der abgegebenen Stromstärke.

Erfindungsgemäß wird unter einer Nadel ein länglicher (vorzugsweise zylindrischer) Körper verstanden, dessen Länge im Verhältnis zum Durchmesser groß ist. Vorzugsweise hat eine Nadel ein zugespitztes Ende, insbesondere ein konisch zugespitztes. Die Nadel oder Nadeln zur Verwendung als erste Elektrode ist/sind vorzugsweise so ausgebildet, dass durch ihre Anwendung der menschliche oder tierische Körper nicht verletzt wird. Der Durchmesser eines einzustechenden Bereichs liegt (ohne Berücksichtigung eines zugespitzten Endes) vorzugsweise zwischen 0,1 und 0,8 mm, bevorzugt zwischen 0,2 und 0,4 mm und insbesondere bei etwa 0,3 mm, wobei ein der einzustechende Bereich vorzugsweise außerdem ein zugespitztes Ende hat; die Länge eines einzustechenden Bereichs liegt vorzugsweise zwischen 10 und 100 mm, bevorzugt zwischen 20 und 50 mm und insbesondere bei etwa 30 mm. Der Durchmesser in einem Griffbereich kann beispielsweise etwa 1-3 mm betragen, um eine einfache Verbindung mehrerer Nadeln zu ermöglichen. Bevorzugte Nadeln haben die Form bekannter Akupunkturnadeln und folgende Dimensionen: 0,2 x 15 mm, 0,25 x 40 mm, 0,3 x 30 mm, 0,3 x 100 mm, 0,35 x 50 mm.

Das Material von Nadel(n) zur Verwendung als erste Elektrode ist vorzugsweise Metall. Bevorzugte Metalle sind Edelstähle, d.h. unlegierte oder legierte Stähle mit geringem Schwefel- und Phosphorgehalt. Weitere Legierungsbestandteile sind vorzugsweise Chrom (vorzugsweise in einem Anteil von 10,5-13 Gew.-% oder höher), Nickel (vorzugsweise in einem geringen Anteil, etwa maximal 10 Gew.-%), Molybdän, Titan und/oder Niob. Bevorzugt ist 18/10 Chrom-Nickel-Stahl oder medizinischer Edelstahl. Bevorzugte Stähle sind solche, die gegen Wasser und schwache organische und anorganische Säuren beständig sind. Insbesondere bevorzugt sind nichtrostende Stähle. Weitere bevorzugte Metalle sind Silber, Gold und Platin. Optional sind die Nadeln lediglich versilbert, vergoldet bzw. platiniert. Bevorzugt sind auch Sinterwerkstoffe, beispielsweise aus Silber/Silberchlorid.

Vorzugsweise umfasst die erste Elektrode eine Mehrzahl von Nadeln, vorzugsweise 3-12, bevorzugt 4-10, 6-10, 6-8 und insbesondere 8. Dies erlaubt ein besonders gutes Umkreisen eines zu behandelnden Bereichs. In besonderen Ausführungsformen kann die erste Elektrode auch eine höhere Anzahl von Nadeln umfassen, was insbesondere vorteilhaft ist, falls mehr als ein zu behandelnder Bereich vorliegt.

In der Gleichstromabgabevorrichtung sind im Fall der Verwendung einer Mehrzahl von Nadeln als erste Elektrode die Spitzen der Nadeln vorzugsweise entlang eines im Wesentlichen kreisförmigen oder elliptischen Umfangs angeordnet. Vorzugsweise liegt die zweite Elektrode außerhalb des von den Nadeln umschriebenen Bereichs. Es ist bevorzugt, dass die elektrisch leitende Verbindung der Nadeln entlang des (kreisförmigen oder elliptischen) Umfangs ausgebildet ist, so dass die Schaltung der Nadeln der ersten Elektrode eine geordnete Reihenschaltung ist und keine Zickzack-Schaltung. Diese Ausführungsformen ermöglichen besonders vorteilhafte Behandlungen.

Alternativ dazu können die Nadeln auch entlang einer im Wesentlichen geraden Linie angeordnet sein.

In besonderen Ausführungsformen können auch eine erste Gruppe von Nadeln und eine oder mehrere weitere Gruppe(n) von Nadeln vorgesehen sein (etwa zwei, drei, vier oder fünf Gruppen von Nadeln als erste Elektrode), was eine Behandlung mehr als eines zu behandelnden Bereichs erlaubt. In diesen Ausführungsformen ist es bevorzugt, wenn die Spitzen der Nadeln der ersten Gruppe entlang eines im Wesentlichen kreisförmigen oder elliptischen Umfangs angeordnet sind und die Spitzen der Nadeln der weiteren Gruppe(n) (jeweils) ebenfalls entlang eines im Wesentlichen kreisförmigen oder elliptischen Umfangs angeordnet sind. Die zweite Elektrode liegt hierbei vorzugsweise außerhalb der von den Nadeln umschriebenen Bereiche. Bevorzugt ist es, wenn die elektrisch leitende Verbindung der Nadeln entlang des jeweiligen (kreisförmigen oder elliptischen) Umfangs der einzelnen Gruppen von Nadeln ausgebildet ist (Reihenschaltung der Nadeln der jeweiligen Gruppe) und/oder die Gruppen durch jeweils eine einzelne elektrisch leitende Verbindung in Reihe geschaltet sind.

Alternativ dazu können die Spitzen der Nadeln der ersten Gruppe entlang einer im Wesentlichen geraden Linie angeordnet sein und die Spitzen der Nadeln der weiteren Gruppe(n) (jeweils) ebenfalls entlang eines einer im wesentlichen geraden Linie, wobei vorzugsweise die Nadeln der einzelnen Gruppen jeweils in Reihe geschaltet sind und/oder die Gruppen durch jeweils eine einzelne elektrisch leitende Verbindung in Reihe geschaltet sind.

Vorzugsweise ist die erste Elektrode (Elektrode zur Verwendung im zu behandelnden Bereich, vorzugsweise Nadelelektrode) als Kathode (negativer Pol) und die zweite Elektrode als Anode (positiver Pol) ausgebildet. Hierdurch werden die Behandlungsmöglichkeiten unter Einsatz der erfindungsgemäßen Gleichstromabgabevorrichtung optimiert.

Gemäß einer alternativen Ausführungsform kann die erste Elektrode allerdings auch als flächige Elektrode (vorzugsweise als Klebeelektrode) ausgebildet sein (optional als Mehrzahl von flächigen Gebilden, beispielsweise zwei, drei, vier oder fünf). Für bevorzugte Ausgestaltungen einer flächigen ersten Elektrode gilt das im Folgenden für eine flächige zweite Elektrode ausgeführte (Variante A) Soweit die zweite Elektrode ebenfalls als flächige Elektrode ausgebildet ist, ist es dabei bevorzugt, wenn die (Gesamt-)Fläche der zweiten Elektrode größer als die (Gesamt-)Fläche der ersten Elektrode ist, beispielsweise um mindestens 50%, 100%, 200%, 400%, 1000% oder 5000% größer. Bevorzugte Größen einer flächigen ersten Elektrode sind 0,5 bis 5 cm², insbesondere 1 cm², bis 2 cm².

Die zweite Elektrode ist vorzugsweise als flächige Elektrode ausgebildet (Variante A). Eine solche Elektrode ist als Oberflächenelektrode einsetzbar und ist vorzugsweise angepasst an eine Anbringung auf der Körperoberfläche, beispielsweise durch Ausgestaltung als Klebeelektrode (wie oben definiert). Bevorzugt ist eine Elektrode, die in einem Klebestreifen eingearbeitet ist oder anderweitig mit einem Klebestreifen verbunden ist. Eine Klebewirkung kann auch durch Elektrodengel oder Elektrodenpaste vermittelt werden. Vorzugsweise ist das Material für die zweite Elektrode ausgewählt aus der Gruppe bestehend aus leitfähiger Gummi, leitfähiges Textil, leitfähiger Kunststoff, Schwamm (zu tränken mit beispielsweise Wasser oder NaCI-Lösung), Sinterwerkstoff (beispielsweise Silber/Silberchlorid) und Metall (beispielsweise Edelstahl, Silber, Gold und/oder Platin).

Bevorzugte Größen einer flächigen zweiten Elektrode sind 25 cm² bis 200 cm², insbesondere 50 cm² bis 100 cm²,

Ohne an eine bestimmte Theorie gebunden zu sein, wird angenommen, dass eine Verwendung von Silber/Silberchlorid-Elektroden vor allem in chloridhaltigem Medium wie dem Körpermilieu das Kontaktpotenzial (junction potential) am Metall/Elektrolyt-Übergang stabilisiert, so dass die Stromabgabe noch besser kontrollierbar wird.

Die zweite Elektrode umfasst optional eine Mehrzahl von flächigen Gebilden wie oben beschrieben, beispielsweise zwei, drei, vier oder fünf.

Daneben ist es auch möglich, die zweite Elektrode als Nadel auszubilden (Variante B), wobei für bevorzugte Ausgestaltungen dieser Nadel das zu den Nadeln der ersten Elektrode Gesagte gilt.

Besonders bevorzugte Kombinationen aus erster und zweiter Elektrode sind wie folgt: nadelförmige erste Elektrode im Kombination mit nadelförmiger zweiter Elektrode, nadelförmige erste Elektrode im Kombination mit flächiger zweiter Elektrode, flächige erste Elektrode ein Kombination mit nadelförmiger zweiter Elektrode und flächige erste Elektrode im Kombination mit flächiger zweiter Elektrode. Es gilt das oben zu nadelförmigen Elektroden bzw. flächigen Elektroden Gesagte. Insbesondere umfasst beispielsweise eine nadelförmige Elektrode optional eine Mehrzahl von Nadeln (beispielsweise zwei, drei, vier oder fünf) und/oder umfasst eine flächige Elektrode optional eine Mehrzahl von flächigen Gebilden (beispielsweise zwei, drei, vier oder fünf).

Gemäß einer alternativen Ausführungsform sind beide Elektroden in einem einzelnen Gebilde vereint (mehrpolige Nadel oder mehrpoliges flächiges Gebilde). Dies ist beispielsweise vorteilhaft bei der Behandlung eines räumlich eng umgrenzten Gebiets oder bei Patienten, die die Applikation von Elektroden generell schlecht tolerieren. So kann erfindungsgemäß eine Nadel die erste Elektrode und die zweite Elektrode entlang ihrer Längserstreckung hintereinander aufweisen, beispielsweise die Kathode näher am einzustechenden Ende als die Anode oder auch die Anode näher am einzustechenden Ende als die Kathode. Ferner sind die Kathode und/oder die Anode hierbei entweder kontinuierlich oder diskontinuierlich. Beispielsweise kann die Oberfläche der Kathode und/oder der Anode optional als ein oder mehrere Zylindermäntel ausgebildet sein.

Vorzugsweise sind die Elektroden, die Mittel zum Verbinden der Elektroden mit der Gleichstromquelle und/oder die Anschlüsse der Gleichstromquelle entsprechend der Polarität gekennzeichnet, beispielsweise durch Farbe oder Form, Symbole wie + und -, Ziffern oder Buchstaben.

Bevorzugt ist eine physiologische akzeptable Stromstärke bzw. eine die Zellen des Körpergewebes nicht schädigende Stromstärke. Die maximale Stromstärke des Gleichstroms beträgt bei der bevorzugten Variante A mit flächiger zweiter Elektrode vorzugsweise 2000 µA, bevorzugt 1000, 700, 500, 400, 300, 250, 200, 150 oder 100 µA(bei nadelförmiger oder flächiger erster Elektrode). Bei der Variante B mit einer Nadel als zweite Elektrode beträgt die maximale Stromstärke des Gleichstroms vorzugsweise 1000, 750, 500, 250, 200, 150, 100, 50, 25 oder 5 µA(bei nadelförmiger oder flächiger erster Elektrode). Sowohl bei Variante A als auch bei Variante B gilt, dass die höchsten der genannten Stromstärken bevorzugt bei flächiger und weniger bei nadelförmiger erster Elektrode ausgewählt werden. Die bevorzugte minimale Stromstärke des Gleichstroms liegt (bei nadelförmiger oder flächiger erster Elektrode) bei 10, 20, 30, 40 oder 50 µA (Variante A) oder 1, 1,5, 2 oder 2,5 µA (Variante B). Besonders bevorzugt sind (bei nadelförmiger oder flächiger erster Elektrode) Bereiche für die Stromstärke von 10-250 µA, 20-250 µA, 10-200 µA, 20-200 µA, 10-150 µA, 20-150 µA, 30-150 µA, 20-100 µA, 30-100 µA, 40-100 µA und 50-100 µA (Variante A) bzw. Bereiche von 1-25 µA, 1,5-20 µA, 2-15 µA, 2-10 µA und 2,5-5 µA (Variante B). Die erfindungsgemäße Gleichstromabgabevorrichtung umfasst vorzugsweise ein Mittel zum Einstellen der Stromstärke und insbesondere ein Mittel zum Einstellen einer minimalen und/oder maximalen Stromstärke, in allen Fällen vorzugsweise fernsteuerbar.

Die Stromdichte, definiert als abgegebene Stromstärke, bezogen auf die von einer Nadel kontaktierte Fläche, beträgt vorzugsweise maximal 10 µA/mm², bevorzugt maximal 7 µA/mm², maximal 5 µA/mm², maximal 3 µA/mm², maximal 2,5 µA/mm², maximal 2 µA/mm², maximal 1,5 µA/mm², maximal 1 µA/mm² oder maximal 0,5 µA/mm². Die elektrische Spannung beim Einsatz der Gleichstromabgabevorrichtung zur Behandlung des menschlichen oder tierischen Körpers beträgt vorzugsweise maximal 5 V, 4,8 V, 4,5 V, 4 V, 3,6 V, 3 V, 2,5 V, 2,4 V, 2 V, 1,5 V oder 1,2 V. Hierdurch wird sichergestellt, dass schädliche Einwirkungen auf den Körper vermieden werden. Die erfindungsgemäße Gleichstromabgabevorrichtung umfasst vorzugsweise ein (insbesondere fernsteuerbares) Mittel zum Einstellen einer maximalen Spannung. Ferner umfasst sie vorzugsweise ein (insbesondere fernsteuerbares) Mittel zum Einstellen einer maximalen Ladung.

Die Stärke des elektrischen Feldes liegt vorzugsweise im Bereich von 10-2500 mV/mm, insbesondere von 200-1500 mV/mm. Die Felddichte kann in der Umgebung nadelförmiger Elektroden noch höher sein, was ein bevorzugtes Behandlungsprinzip unter Verwendung der erfindungsgemäßen Gleichstromabgabevorrichtung ist. Stärke und Verlauf des elektrischen Feldes in der Umgebung nadelförmiger Elektroden richten die Wirkung bei Einsatz der erfindungsgemäßen Gleichstromabgabevorrichtung hauptsächlich in das Gebiet, in dem die Elektrode appliziert ist bzw. in ihre unmittelbare Nachbarschaft. In der unmittelbaren Umgebung einer nadelförmigen Elektrode nimmt die Feldstärke in orthogonaler Richtung exponentiell ab.

Optional umfasst die erfindungsgemäße Gleichstromabgabevorrichtung ferner ein Mittel zur zeitlichen Steuerung, mit dem mehrere Zeiträume zur Abgabe des Gleichstroms vorbestimmt werden können. Im einfachsten Fall ist dies eine elektronische schaltbare Unterbrechung der elektrisch leitenden Verbindung zwischen Kathode, Gleichstromquelle und Anode. Vorzugsweise ist das Mittel zur zeitlichen Steuerung mit einem Mittel zur Einstellung eines minimalen Zeitraums verknüpft, wobei Letzteres vorzugsweise eine Bestimmung eines minimalen Zeitraums von 1 s, 10 s, 1 min, 2 min, 5 min, 10 min, 20 min oder 30 min erlaubt. Vorzugsweise ist das Mittel zur zeitlichen Steuerung fernsteuerbar. Gemäß einer bevorzugten Ausführungsform ist das Mittel zur zeitlichen Steuerung ferner mit einem (vorzugsweise fernsteuerbaren) Mittel zur Bestimmung eines maximalen Zeitraums verknüpft, wobei Letzteres vorzugsweise eine Bestimmung eines maximalen Zeitraums von 2 h, 1 h, 50 min, 40 min, 30 min, 20 min, 10 min, 5 min oder 2 min erlaubt.

Vorzugsweise umfasst die erfindungsgemäße Gleichstromabgabevorrichtung ein (insbesondere fernsteuerbares) Mittel zum Auflaufen und Auslaufen (Ramping) der Stromstärke. Ein solches Mittel erlaubt zu Beginn der Behandlung ein Ansteigen der Stromstärke von Null bis zum Sollwert innerhalb eines vorbestimmbaren Zeitraums (mit einer bevorzugten Länge von 1-60 Sekunden, besonders bevorzugt 5-45 Sekunden und insbesondere 10-30 Sekunden) und am Ende der Behandlung ein Abfallen vom Sollwert bis auf Null innerhalb eines vorbestimmbaren Zeitraums (mit einer bevorzugten Länge von 1-60 Sekunden, besonders bevorzugt 5-45 Sekunden, weiter bevorzugt 10-30 Sekunden und insbesondere 15 Sekunden). Ein langsames Auflaufen und Auslaufen der Stromstärke ist vorteilhaft, weil sonst - beim sprunghaften An- oder Ausschalten des Stromes - das behandelte Individuum ein unangenehmes Zucken oder ein Stromschlaggefühl verspürt.

Optional umfasst die erfindungsgemäße Gleichstromabgabevorrichtung ein (vorzugsweise fernsteuerbares) Mittel zum Umschalten der Polarität der Elektroden während einer Behandlung. Dieses ist vorzugsweise mit einem Mittel zur zeitlichen Steuerung verknüpft, so dass es beispielsweise möglich ist, die Polarität jede Sekunde, alle 10 s, jede Minute, alle 2 min, alle 5 min oder alle 10 min umzuschalten.

Die Gleichstromabgabevorrichtung ist optional auf einen Testmodus einstellbar, in dem eine konstante Spannung von etwa 1-8 V, 2-6 V oder 3-5 V abgegeben wird. Damit kann überprüft werden, ob die Elektroden (beispielsweise insbesondere alle Nadeln) elektrisch korrekt verkoppelt sind oder nicht, oder ob in irgendeinem Kabel ein okkulter Kabelbruch vorliegt. Vorzugsweise umfasst die Gleichstromabgabevorrichtung einen Signalgeber (etwa einen Tonerzeuger), der anzeigt, wenn ein korrekter Stromfluss entsteht. Aus einer Abwesenheit des Signals kann geschlossen werden, dass die Kopplerkette unterbrochen ist.

Ein Signalgeber ist vorzugsweise auch dafür einsetzbar, den Anfang und/oder das Ende der Behandlung anzuzeigen. Ferner ist ein Signalgeber vorzugsweise dafür einsetzbar anzuzeigen, falls während einer Behandlung der Stromfluss unterbrochen wird oder die Impedanz des Patienten zu hoch ist, insbesondere in Kombination mit einem Abschaltmittel.

Mit Hilfe des Testmodus können auch die einzelnen Elektroden (z.B. Nadeln) direkt stimuliert werden und aus der Reaktion des Patienten (Muskelzuckung bzw. in nicht kontraktilem Gewebe Schmerz) auf die richtige Positionierung der Elektrode (insbesondere Nadel) geschlossen werden, wie unten näher beschrieben.

Das erfindungsgemäße Kit zum Herstellen einer Gleichstromabgabevorrichtung (zweiter Aspekt der vorliegenden Erfindung) umfasst vorzugsweise zusätzlich eine Anleitung zur therapeutischen oder kosmetischen Behandlung des menschlichen oder tierischen Körpers, wobei die Behandlung vorzugsweise ist wie im Folgenden genauer erläutert.

Eine bevorzugte Ausführungsform der erfindungsgemäßen Gleichstromabgabevorrichtung umfasst eine Gleichstromquelle, eine erste Elektrode sowie eine zweite Elektrode zum Verbinden mit der Gleichstromquelle, ein Mittel zum Konstanthalten der Stromstärke bei der Abgabe des Gleichstroms, eine erste Elektrode, die als Nadel oder Mehrzahl miteinander elektrisch leitend verbundener Nadeln ausgebildet ist und eine zweite Elektrode, die als flächige Elektrode ausgebildet ist. Bevorzugt ist dabei eine maximale Stromstärke des Gleichstroms von 2000 µA, weiter bevorzugt 1000 µA, besonders bevorzugt 700 µA und insbesondere 500 µA. Selbstverständlich sind auch die oben aufgeführten niedrigeren Stromstärken mit dieser Ausführungsform kombinierbar.

Die erfindungsgemäße Gleichstromabgabevorrichtung wird vorzugsweise bei der Behandlung von Entzündungen und/oder Schmerzen, insbesondere der Muskeln, Nerven, Sehnen oder Knochen eingesetzt.

Bei der Behandlung ist eine Applikation von Dauerstrom gegenüber einer Applikation von gepulstem Strom bevorzugt. Alternativ möglich sind eine undulierende Stromstärke wie oben beschrieben oder eine sich nicht periodisch verändernde Stromstärke (die zu jedem Zeitpunkt im Wesentlichen den gleichen Wert hat). Vorzugsweise wird die Stromstärke bei der Abgabe des Gleichstroms (oder der Wert, um den die Stromstärke unduliert) konstant gehalten, insbesondere auch in dem Fall, dass sich ein zwischen den Elektroden anliegender Widerstand verändert.

Die Dauer einer bevorzugten Behandlung liegt zwischen 1 min und 2 h, 5 min und 1 h, 10 min und 50 min, 20 min und 40 min und vorzugsweise bei 30 min. Bevorzugt umfasst die Behandlung eine Gesamtstromabgabezeit von 60 min, 45 min, 30 min oder 20 min. Vorzugsweise erfolgt die Stromabgabe bei der Behandlung ohne Unterbrechung. Gemäß einer alternativen Ausführungsform kann die Behandlung jedoch auch mehrere vorbestimmte Zeiträume (beispielsweise 2, 3, 4, 5, 6 oder mehr vorzugsweise gleich lange Zeiträume) umfassen, während derer der Gleichstrom appliziert wird, wobei zwischen den Zeiträumen vorzugsweise eine Pause von 1 s bis 5 min, 10 s bis 3 min oder 30 s bis 1 min vorgesehen ist. Alternativ kann ein An- und Abschalten des Gleichstroms mit einer Frequenz von 0,01-1 Hz, vorzugsweise 0,02 bis 0,2 Hz und insbesondere 0,05 bis 0,1 Hz erfolgen. Diese Zeiträume bzw. das An- und Abschalten werden gesteuert durch das gegebenenfalls vorgesehene Mittel zur zeitlichen Steuerung (siehe oben). Vorzugsweise erfolgt zu Beginn und am Ende der Behandlung ein langsames Auf- bzw. Auslaufen (Ramping) der Stromstärke, etwa über einen Zeitraum von jeweils 1-60 Sekunden, bevorzugt 5-45 Sekunden und insbesondere 10-30 Sekunden und besonders bevorzugt 15 Sekunden.

Die Behandlung erfolgt vorzugsweise innerhalb eines Tages, insbesondere innerhalb eines Zeitraums von 4, 3 oder 2 Stunden. Je nach Behandlungsansatz ist es bevorzugt, die Behandlung einmalig oder wiederholt (insbesondere ein-, zwei- oder dreimal pro Woche oder täglich) durchzuführen.

Im Vorfeld der Stromapplikation wird der zu behandelnde schmerzende/entzündete Bereich vorzugsweise eingekreist und so lokalisiert, bevorzugt durch tiefes Tasten bis zum Knochen von allen Seiten her. In der Terminologie der TCM wird ein entsprechender druckschmerzhafter Punkt "Ahshi" genannt. Der zu behandelnde Bereich wird nach optionalem Desinfizieren mit einer Elektrode versehen, bevorzugt einer oder mehreren Nadeln (vorzugsweise durch Einstecken) und insbesondere mit einer Mehrzahl von Nadeln umschrieben (insbesondere kreisförmig oder elliptisch). Umschreiben bedeutet hierbei vorzugsweise, dass die Nadeln entlang des Randes dieses Bereichs gesteckt werden. Weitere Möglichkeiten bestehen darin, die Nadeln einige Millimeter außerhalb des Randes oder aber im Inneren des Bereichs zu stecken. Das genannte Einstecken der Nadel(n) erfolgt vorzugsweise möglichst tief (zum Beispiel in die Subkutis, in einen Muskel, in die Ligamente oder Sehnen, unter das Acromion, auf die Facetten der Wirbelsäule oder auf die Knochenhaut), optional nach Lokalanästhesie. Alternativ dazu kann der zu behandelnde Bereich auch durch Anordnen einer Mehrzahl von Nadeln entlang einer im Wesentlichen geraden Linie mit Nadeln versehen werden, wobei die Linie diesen Bereich schneidet oder tangieret oder auch außerhalb des Bereichs liegt.

Die zweite Elektrode wird am oder im Körper positioniert, und zwar soweit die erste Elektrode mehrerer Nadeln umfasst vorzugsweise außerhalb eines davon umschriebenen Bereichs. Insbesondere wird die zweite Elektrode in einem anderen Körperbereich als die erste Elektrode positioniert. Vorzugsweise wird die zweite Elektrode (Variante A: flächige Elektrode) oberhalb von großen Muskelgruppen oder Fettschichten positioniert, damit nicht einzelne Nerven durch sie stimuliert werden. Eine zweite Elektrode gemäß Variante B (nadelförmige Elektrode) wird vorzugsweise intramuskulär appliziert.

Vorzugsweise wird vor der eigentlichen Behandlung durch kurzzeitiges Anlegen eines Stroms sichergestellt, dass (eine) eingesteckte Nadel(n) nicht in der Nähe von Nervenwurzeln liegen, damit es bei der Behandlung nicht zu einer Schmerzreaktion oder einer motorischen Reaktion kommt. Andernfalls müsste(n) die Nadel(n) etwas zurückgezogen oder an einer anderen Stelle positioniert werden.

Alternativ dazu kann in bestimmten Ausführungsformen ein Muskelzucken auch gezielt genutzt werden, um die Positionierung einer Nadel zu überprüfen. Durch elektrischen Strom kann ein Muskelzucken ausgelöst werden, beispielsweise indem Elektroden während einer Stromabgabe an eingestochene Nadeln gehalten werden. Je stärker dieses Zucken, desto besser ist die Nadel positioniert. Wie oben bereits erwähnt, ist es möglich, etwa unter Verwendung des beschriebenen Testmodus die einzelnen Elektroden (z.B. Nadeln) direkt zu stimulieren und aus der Reaktion des Patienten auf die richtige Positionierung der Elektrode (insbesondere Nadel) zu schließen. So bewirkt eine in einen entzündeten schmerzhaften Muskel eingesetzte Nadel bei direkter Teststimulation eine im Vergleich zu einer in einen nicht entzündeten Muskel eingestochenen Nadel eine verstärkte Muskelzuckung. Bei in nicht kontraktiles Gewebe eingestochenen Nadeln verspürt der Patient bei direkter Teststimulation einen vermehrten Schmerz (Brennen) an der Nadel im entzündeten Gewebe im Vergleich zu einem nicht entzündeten Gewebe.

Optional können auch mehrere separat regelbare Stromquellen eingesetzt werden.

Es ist möglich, dass die durch die erfindungsgemäße Gleichstromabgabevorrichtung erzeugten elektrischen Felder zusätzlich zu ihrer antiphlogistischen und analgetischen Wirkung eine rekonstruierende Wirkung aufweisen, beispielsweise über eine Förderung des Gefäßwachstums, unter anderem über die Ausschüttung von VEGF und einen Einfluss auf Endothelzellen. Weiterhin ist es möglich, dass sie zu einer Bewegung und Neuanordnung von Zellmembranrezeptoren führen, die Teilungsrate von bestimmten Zellen erhöhen, die Zellwanderung von Epithelzellen (insbesondere zur Kathode hin) und die Wundheilung beschleunigen. Auch ist es denkbar, dass dadurch die periphere Nervenregeneration nach Rückenmarkstrauma durch Wachstum zur Kathode hin beschleunigt werden kann, die dann vorzugsweise kopfwärts liegen würde.

Ohne an eine bestimmte Theorie gebunden zu sein, ist die Wirkung der Anwendung der erfindungsgemäßen Gleichstromabgabevorrichtung auf den menschlichen oder tierischen Körper zurückzuführen auf die unmittelbare Einwirkung des verabreichten elektrischen Stroms bzw. des angelegten elektrischen Feldes auf das betroffene Gewebe bzw. die betroffenen Zellen. Eine Erklärung ist beispielsweise eine Veränderung der elektrischen Erregbarkeit von Zellen, insbesondere Nervenzellen (De- oder Hyperpolarisation), womöglich über eine Wirkung auf Kationenkanäle oder über eine temporäre Verschiebung des lonengleichgewichts zwischen Intrazellulär- und Extrazellulärraum. Hierfür käme insbesondere ein Efflux von Kaliumionen und anderen Kationen in den Extrazellulärraum infrage, wodurch eine lokale antiphlogistische und analgetische Wirkung erklärt werden könnte. Auch eine Regeneration von aseptischen Wunden oder degenerativ veränderten Gewebeanteilen oder eine Wanderung von Zellen im elektrischen Feld könnte (mit)ursächlich für die beobachtete Wirkung sein. Es wird angenommen, ohne an eine Theorie gebunden zu sein, dass der verabreichte elektrische Strom bzw. das angelegte elektrische Feld fundamentale Entzündungsprozesse, Prozesse der Schmerzentstehung und/oder der Geweberegeneration in den Zellen und im Gewebe direkt und lokal beeinflusst. Dadurch werden grundlegende elektrophysiologische/neurophysiologische Mechanismen beeinflusst.

Die Stromstärke und Spannung sind dabei um ein Vielfaches (mehrere Zehnerpotenzen) kleiner als bei bekannten Elektroakupunkturgeräten. Insbesondere wird Gleichstrom und nicht Wechselstrom eingesetzt.

Bei bekannten medizinischen Elektrostimulationsgeräten beruht das Wirkprinzip auf einer hohen Intensität der Spannung und/oder des Stroms. Zum Beispiel soll bei galvanischen Bädern oder bei der Kauterisation eine Erhitzung des Gewebes bewirkt werden, oder bei der TENS (Gate Gontrol Theorie) eine überschwellige Rezeptorreizung zur Schmerzunterdrückung. Im Gegensatz hierzu arbeitet die erfindungsgemäße Gleichstromabgabevorrichtung bei kleinsten Spannungen, Stromstärken und elektrischen Feldern, wodurch insbesondere eine entzündungshemmende, schmerzhemmende und/oder regenerative Wirkung erzeugt wird.

Nach Einstechen einer Nadel wird zwischen dem negativen Pol an der Nadel und einer auf der Haut aufgeklebten breitflächigen Elektrode beispielsweise eine Potenzialdifferenz von 100-300 mV gemessen. Durch schnelles manuelles Drehen der Nadel kann sich die Potenzialdifferenz erhöhen, was in erster Linie auf die Beeinflussung des Elektroden-Kontaktpotenzials zurückzuführen ist und nachfolgend logarithmisch wieder auf den Ausgangswert absinken. Das Einstechen von Nadeln und ihre manuelle Stimulation sind grundlegende Techniken der analgetischen Akupunktur.

Gegenstand der vorliegenden Erfindung ist ferner die erfindungsgemäße Gleichstromabgabevorrichtung zur Anwendung bei der Behandlung folgender Beeinträchtigungen oder Beschwerden: Migräne, Spannungskopfschmerz (z.B. migräneartiger Spannungskopfschmerz), Neuralgien (z.B. post-Zoster-Neuralgie, Occipitalisneuralgie, Trigeminusneuralgie, Neuralgie des Nervus femoralis, besonders postoperativ), Herpes Zoster-Schmerz, neuropathischer post-Zoster Schmerz, Bing-Horton-Syndrom, Tinnitus, Allergien, entzündliche Zeichen bei Allergie, HWS-Syndrom, BWS-Syndrom, LWS-Syndrom, chronischer Kreuzschmerz, Spinalkanalstenose, Zervikobrachialgie, Ischialgie, Radikulitis, Periarthritis humero scapularis, Schmerzen bei Arthrose, Gonarthrose, Arthritis (soweit nicht systemisch bedingt), Tendinitis (wie Tennisarm, Golfersarm (Epikondylitis lateralis-, medialis), Tendovaginitis, Ansatztendinosen, Achillodynie, Fersensporn, Hautrötung, Hautentzündung, Seborrhöe, Psoriasis, seborrhoische, erythematöse und/oder psoriatische Erscheinungen, Akne, Haarausfall (z.B. Alopezie), durch lokale Reizzustände verursachte Bewegungseinschränkungen vor allem der Haut wie lokale Verhärtungen und Spannungen.

Entsprechendes gilt für die Verwendung der erfindungsgemäßen Gleichstromabgabevorrichtung zur Behandlung der genannten Indikationen sowie für die Verwendung der erfindungsgemäßen Gleichstromabgabevorrichtung zur Herstellung einer therapeutischen Vorrichtung zur Behandlung der genannten Indikationen.

Die erfindungsgemäße Gleichstromabgabevorrichtung erzeugt vorzugsweise eine langfristige, sich von Behandlung zu Behandlung aufbauende regenerative Wirkung, beispielsweise bei chronischen Tendinosen oder chronischen Neuropathischeschmerzen.

Vorzugsweise sind folgende Behandlungen ausgeschlossen: Behandlung eines Haarfollikels, Behandlung offener Wunden und Behandlung einer Hautschädigung.

Gemäß einem weiteren Aspekt betrifft die vorliegende Erfindung die Verwendung der erfindungsgemäßen Gleichstromabgabevorrichtung zur kosmetischen Behandlung des menschlichen oder tierischen Körpers.

Außerdem betrifft die vorliegende Erfindung gemäß einem weiteren Aspekt ein Verfahren zum Herstellen einer Gleichstromabgabevorrichtung umfassend die folgenden Schritte: Bereitstellen eines erfindungsgemäßen Kits (gemäß dem dritten Aspekt der vorliegenden Erfindung), Bereitstellen einer Gleichstromquelle bzw. - falls das Kit eine Batterie als Gleichstromquelle enthält - Bereitstellen der Gleichstromquelle, Bereitstellen der flächigen Elektrode oder der Nadel oder elektrisch leitendes Verbinden der Mehrzahl von Nadeln zum Bilden der ersten Elektrode (wobei die Spitzen einer Mehrzahl von Nadeln vorzugsweise entlang eines im Wesentlichen kreisförmigen oder elliptischen Umfangs oder entlang einer im Wesentlichen geraden Linie angeordnet werden und die elektrisch leitende Verbindung vorzugsweise entlang des Umfangs erfolgt), Bereitstellen der flächigen Elektrode oder der Nadel oder elektrisch leitendes Verbinden der Mehrzahl von Nadeln zum Bilden der zweiten Elektrode (wobei die Spitzen einer Mehrzahl von Nadeln vorzugsweise entlang eines im Wesentlichen kreisförmigen oder elliptischen Umfangs oder entlang einer im Wesentlichen geraden Linie angeordnet werden und die elektrisch leitende Verbindung vorzugsweise entlang des Umfangs erfolgt), Verbinden der ersten Elektrode mit der Gleichstromquelle und Verbinden der zweiten Elektrode mit der Gleichstromquelle.

Ein weiterer Aspekt der vorliegenden Erfindung ist die erfindungsgemäße Gleichstromabgabevorrichtung zur Anwendung in einem Verfahren zur therapeutischen Behandlung des menschlichen oder tierischen Körpers bzw. die Verwendung der erfindungsgemäßen Gleichstromabgabevorrichtung in einem Verfahren zur therapeutischen Behandlung des menschlichen oder tierischen Körpers.

Schließlich betrifft die vorliegende Erfindung ein durch die erfindungsgemäße Gleichstromabgabevorrichtung erzeugbares elektrisches Feld. Das Feldmaximum liegt hierbei um den Nadelkorpus und eine Nadelspitze.

Weiterhin betrifft die vorliegende Erfindung ein solches elektrisches Feld zur Anwendung in einem Verfahren zur therapeutischen Behandlung des menschlichen oder tierischen Körpers, insbesondere ein solches elektrisches Feld zur Behandlung von Entzündungen und/oder Schmerzen, insbesondere der Muskeln, Nerven, Sehnen oder Knochen oder zur Behandlung einer der oben genannten Indikationen.

### Beispiele

### Beispiel 1: Clusterkopfschmerz

Männlicher Patient, 56 Jahre. Seit 3 Jahren bekanntes Bing-Horton-Syndrom mit in unregelmäßigen Abständen nahezu unerträglichen Gesichtsschmerz-Attacken. Dies ist ein seltenes Krankheitsbild mit extremsten Schmerzen - im Englischen auch als "Suicidal Headache" bezeichnet, bei dem bisher bekannte Therapieverfahren überhaupt nicht befriedigend sind. Bei Vorstellung täglich, teils mehrmals, unerträgliche Schmerzanfälle. Bisherige Therapien alle ohne Erfolg (u.a. Analgetika, Sauerstoffinhalation). Behandlung mit der erfindungsgemäßen Gleichstromabgabevorrichtung: Klebe-Oberflächenelektrode (Klebepad) Oberarm als Anode, 4 Nadeln 0,2 x 15 mm als Kathode unterhalb des Auges und 1 cm neben dem Nasenflügel links, Nadeln kreisförmig gesteckt und entlang des Kreises elektrisch leitend verbunden. Stromstärke 250 µA, 35 Minuten. Sofort nach der ersten Behandlung Reduktion der Anfallshäufigkeit von zweimal täglich auf einmal in vier Tagen, Schmerzintensität reduziert um 40% (VAS). Nach der zweiten Behandlung vollständiges Sistieren der Schmerzattacken. Der Patient ist bis heute beschwerdefrei, Nachbeobachtung 4 Monate.

### Beispiel 2: HWS Syndrom mit Ausstrahlung in den 6. Halsnerven (Radikulitis C6)

Männlicher Patient, 72 Jahre. Seit 4 Monaten starke Schmerzen im HWS Bereich li, tags und nachts ausstrahlend - mit Kribbelparästhesien und Schmerzen - in den linken Nacken, Oberarm, Unterarm bis Daumen li. Bisherige Therapie mit Analgetika, Salben, Injektionen ohne Besserung. Therapie mit Gleichstromabgabevorrichtung: Klebepad Bauchbereich links, 8 Nadeln 0,3 x 30 mm 3 cm paravertebral C5-C6 li, Nadeln kreisförmig gesteckt, Stromstärke 230 µA, 20 Minuten. Erste Schmerzlinderung 90 Minuten nach Therapie. Zweite Behandlung nach 4 Tagen. Danach lassen die Restbeschwerden sofort nach und der Patient ist beschwerdefrei. Nachbeobachtung 5 Monate.

### Beispiel 3: Fußschmerz, Morton-Neuralgie

Weiblicher Patient, 61 Jahre. Seit 1 Jahr stärkste Schmerzen an der 4. Zehe li. Im Kernspin nachgewiesene Auftreibung des Interdigitalnerven. Diagnose Morton-Neuralgie. Bei der Morton-Neuralgie sind bisher bekannte konventionelle konservative und operative Therapieverfahren meist völlig unbefriedigend, die Patienten haben einen sehr hohen Leidensdruck, da sie nur unter starken Fußschmerzen laufen können. Erfindungsgemäße Behandlung: Klebepad Wade links (Anode), Nadelelektrode (Kathode) 8 Nadeln 0,2 x 15 mm 4. Zehe links, Reihenschaltung, Stromstärke 60 µA, 40 Minuten. Zwei Behandlungen im zeitlichen Abstand von 7 Tagen. Ca. 70% (VAS) Besserung der Schmerzen 1 Tag nach der ersten Behandlung, vollständig schmerzfrei nach der zweiten Behandlung. Nachbeobachtung 1,5 Jahre.

### Beispiel 4: Occipitalis Neuralgie

Weiblicher Patient, 60 Jahre. Skisturz vor 4 Wochen. Seitdem zunehmend Kopfschmerzen rechter Hinterkopf. Injektionen, 3 Akupunkturbehandlungen, nichtsteroidale Analgetika (Diclofenac, Ibuprofen) wirkungslos, zuletzt seit 2 Wochen Tilidin Tropfen. Starker Leidensdruck. Gesamter Hinterkopfbereich rechts mit Ausstrahlung ins rechte Ohr maximal berührungsempfindlich. Entscheidung zur erfindungsgemäßen Therapie mit Gleichstromabgabevorrichtung: Klebepad Oberarm rechts, 8 Nadeln 0,25 x 40 mm im maximal schmerzhaften und druckempfindlichen Bereich Hinterhaupt und paravertebral C1-C2 rechts, Nadeln in Reihe verbunden, Stromstärke 125 µA, 30 Minuten. Erste Besserung 60 Minuten nach Therapie. 3 Stunden später vollständig schmerzfrei. Seitdem beschwerdefrei, Nachbeobachtung 2 Monate. Occipitalis Neuralgien (wie bei dieser Patientin) können generell nach akuten Traumen entstehen. Bei dieser Patientin waren die Schmerzen ungewöhnlich stark. Eine Chronifizierung (wie sonst häufig bei Occipitalis Neuralgien) konnte verhindert werden.

### Beispiel 5: Tendinose Ellbogen "Golfersarm"

Männlicher Patient, 45 Jahre. Ellbogen medial punktuell. Bisherige Therapie 9 Kortisoninjektionen, viermal Stoßwelle, Physiotherapie, 10 Akupunkturbehandlungen ohne anhaltenden Erfolg. Erfindungsgemäße Therapie: Klebepad Oberarm rechts, Lokalanästhesie rechter Ellbogen, 8 Nadeln 0,3 x 30 mm als Elektrode im maximal schmerzhaften und druckempfindlichen Bereich kreisförmig angeordnet. 270 µA. 30 Minuten. Erste Besserung 12 Stunden nach Ende Behandlung, ab dem zweiten Tag danach beschwerdefrei. Anhaltend beschwerdefrei, Nachbeobachtung 4 Monate. Überraschende Effizienz mit nur einer einzigen Behandlung.

### Beispiel 6: Akuter Kreuzschmerz mit Ischialgie

Weiblicher Patient, 58 Jahre. 4 Tage lang LWS Schmerz rechts, Punktum Maximum über den Akupunkturpunkten BI 25 und Gb 30, ausstrahlend entlang des Blasenmeridians in den Unterschenkel. Behandlung mit der erfindungsgemäßen Gleichstromabgabevorrichtung: als Anode Klebepad Abdomen Flanke links, Lokalanästhesie, Ellbogen, als Kathode: 8 Nadeln 0,3 x 100 mm im maximal schmerzhaften und druckempfindlichen Bereich der Gesäßmuskulatur, 8 weitere Nadeln im schmerzhaften Bereich der Wade, elektrische Verkopplung aller Nadeln. 250 µA. Direkt nach der Behandlung nahezu schmerzfrei, vollständig beschwerdefrei am nächsten Tag.

### Beispiel 7: Chronischer Kreuzschmerz bei Wirbelkanalstenose

Weiblicher Patient, 78 Jahre. Über 1,5 Jahre trotz konventioneller fachorthopädischer Therapie chronischer Kreuzschmerz rechts mehr als links, verstärkt durch längeres Sitzen. Im Kernspin erhebliche Einengung des Spinalkanals darstellbar. 15 Akupunkturbehandlungen ohne maßgebliche Besserung. Erfindungsgemäße Therapie: Klebepad Abdomen Flanke rechts, Lokalanästhesie paravertebral von L4-L5, 8 Nadeln 0,3 x 100 mm im maximal schmerzhaften und druckempfindlichen Bereich paravertebral L4-L5, vorschieben bis Knochenkontakt zu den kleinen Wirbelgelenken, 4 Nadeln werden daran vorbei bis in die Nähe der Nervenaustritte vorgeschoben. Elektrische Verkopplung aller Nadeln. 250 µA. Nach erster Behandlung ca. 20% (VAS) Besserung des Kreuzschmerzes, nach der zweiten Behandlung ca. 60% (VAS) Besserung, nach der dritten Behandlung ist der Beinschmerz komplett verschwunden, noch ca. 10% (VAS) Kreuzschmerz. Zusätzlich zu den erfindungsgemäßen Behandlungen erfolgten 4 konventionelle Akupunkturbehandlungen.

### Beispiel 8: Spannungskopfschmerz und Migräne

Männlicher Patient, 48 Jahre. Seit 15 Jahren Kopfschmerzen, ca. 2-8 Tage / Monat. Zweimal pro Monat mit Übelkeit, klopfendem, pulsierenden Schmerz einseitig meist rechts. Bisherige Therapie mit Indometacin und anderen peripheren Analgetika. Behandlung mit erfindungsgemäßer Gleichstromabgabevorrichtung bei akutem Migränekopfschmerz. Klebepad Schulter rechts, 4 Nadeln 0,3 x 30 mm in den maximal schmerzhaften und druckempfindlichen Bereich Hinterkopf rechts, sowie 4 weitere Nadeln im Verlauf der Schmerzausstrahlung bis zur Stirn rechts. 125 µA, 30 Minuten. Beginnende Besserung nach 45, kein Kopfschmerz mehr nach 90 Minuten. Wiederholung der gleichen Therapie nur bei akuten Anfällen. Innerhalb von 4 Behandlungen Reduktion der Kopfschmerzhäufigkeit auf ca. 4 / Jahr bei gleichzeitiger Reduktion der Schmerzen auf 30% des Ausgangswertes. Nachkontrolle 3 Jahre.

### Beispiel 9: Akuter Schulterschmerz mit Entzündung der subacromialen Bursa und Supraspinatustendinitis

Weiblicher Patient, 48 Jahre. Nach Überlastung der rechten Schulter bei einem Umzug seit 3 Tagen foudroyant zunehmender Schulterkapselschmerz rechts. Röntgen - Kalkdepots in der subacromialen Bursa. Maximaler Druckschmerz, leichte Rötung über dem tuberculum majus, Schulterbeweglichkeit aufgehoben, zahnschmerzartiger Nachtschmerz in der rechten Schulter. Erfindungsgemäße Therapie: Klebepad mit Anode Flanke rechts, Kathode an 4 Nadeln 0,2 x 15 mm kreisförmig über dem maximalen Schmerz am tuberculum, 4 Nadeln 0,3 x 30 mm von dorsal in den Subacromialraum. 125 µA, Stimulation über 30 Minuten. Beginnende Besserung ca. 2 Stunden nach Therapieende. Nachts ist die Schulter jetzt schmerzfrei. Beweglichkeit in allen Freiheitsgraden (Ante-Retroversion, Abduktion) ca. 50% freier. 2 Tage nach der Therapie Schmerzrezidiv in der Nacht. Patient stellt sich erneut zur Behandlung vor. Gleiches Procedere am tuberculum majus, zusätzlich werden 4 Nadeln 0,25 x 40 mm von verschiedenen Seiten subacromial entlang des Verlaufs der Supraspinatussehne von ventral vorgeschoben, jeweils bis zum Auslösen des typischen Schmerzes. Verkopplung aller Sonden in Serienschaltung. 250 µA, 20 Minuten Stimulation. Erste Schmerzlinderung direkt nach Therapieende. Vier Stunden später deutliche Besserung, schmerzfrei am nächsten Tag. Nach einer Woche komplett freie Schulterbeweglichkeit. Die Patientin ist beschwerdefrei. Nachkontrolle 2 Monate.

### Beispiel 10: Entzündung der Kniegelenkskapsel bei Arthrose

Weiblicher Patient, 73 Jahre. Seit Jahren bekannte Gonarthrose bds., Kellgren Stadium 3-4. Seit 4 Monaten vermehrt Schmerzen nach Belastung und nachts im medialen Kniegelenkkapselbereich links. Nach 6 Akupunkturen Konzentration des Schmerzpunktes am Ansatz des medialen Knieseitenbandes. Erfindungsgemäße Therapie: am Schmerzpunkt kreisförmiger Einstich von 8 Nadeln 0,3 x 30 mm und ringförmige elektrische Verkopplung. Verbindung mit Kathode. Klebepad linker Oberschenkel (Anode), Stimulation 250 µA über 35 Minuten. Ab dem zweiten Tag nach der Behandlung ca. 50% Besserung. Nach einer Woche gleiche Therapie. Zwei Tage später schmerzfrei. Nachbeobachtung 5 Monate.

### Beispiel 11: Retropatelläres Schmerzsyndrom mit Schmerzen am Rectus femoris - Patella Ansatz an beiden Kniegelenken

Männlicher Patient, 34 Jahre, Sei 2 Jahren Schmerzen beim Treppensteigen und längerem Gehen, ausgelöst durch forciertes Bodybuilding-Training. Mehrere Cortison-Injektionen, Krankengymnastik, physikalische Therapie und Akupunkturbehandlungen ohne Erfolg. Erfindungsgemäße Therapie: Klebepad mit Anode an der Flanke, linkes und rechtes Knie unter Lokalanästhesie jeweils acht 0,3 x 30 mm Nadeln, kreisförmig gekoppelt und mit der Kathode verbunden, 260 µA über 30 Minuten. 2 Stunden nach der Behandlung schmerzfreies Treppensteigen möglich, wobei die Schmerzlinderung über 4 Tage anhält, dann erneute Therapie mit gleichem Procedere. Wiedervorstellung nach 2 Wochen, der Patient berichtet über eine ca. 40% anhaltende Besserung, erneute gleiche Therapie, Wiedervorstellung nach 4 Wochen, jetzt insgesamt 70% Besserung, erneute gleiche Therapie, Wiedervorstellung nach 6 Wochen, der Patient ist beschwerdefrei trotz intensivem Bodybuilding-Training. Nachbeobachtung 5 Monate.

### Beispiel 12: Akute Distorsion des Sprunggelenkes

Männlicher Patient, 43 Jahre. Beim Fußball mit dem rechten Fuß umgeknickt. Schwellung und Druckschmerz an typischer Stelle ventrolateral des Außenknöchels, geringe livide Verfärbung durch Hämatom. Der Fuß kann nicht bewegt werden, Schonhinken mit Gehstütze. Erfindungsgemäße Therapie: Unter Lokalanästhesie 8 Nadeln 0,25 x 40 mm direkt in den Schmerzbereich bis an das Periost, Klebepad mit Anode an den Unterschenkel. 30 Minuten Stimulation. 60 Minuten nach Therapieende deutliche Besserung. Weitere Besserung am nächsten Tag. Danach wieder uneingeschränkte Belastbarkeit beim Fußball, trotz noch vorhandener lokaler Schwellung. Anmerkung: in der Regel braucht eine Sprunggelenksdistorsion 3-6 Wochen bis zur Abheilung, interessant ist hier die Beschleunigung des Heilungsvorganges bei einer akuten Sportverletzung.

### Beispiel 13: Akuter Muskelfaserriss rechter Oberschenkel

Beim Fußball trat beim Patienten plötzlicher Schmerz im rechten Oberschenkel auf. Anhaltender Belastungsschmerz, livide Verfärbung über 3 Tage. Im Ultraschall aufgelockerte unregelmäßige Faserstruktur des Muskulus vastus femoris lateralis. Drei Akupunkturbehandlungen ohne maßgebliche Besserung. Weiterhin lokalisierter Druckschmerz und Belastungsschmerz rechter Oberschenkel. Erfindungsgemäße Therapie: 6 Nadeln 0,25 x 40 mm kreisförmig um das Schmerzzentrum, 2 Nadeln zentral in den Locus dolendi, Pad mit Anode rechte Flanke. Kathode an die Nadelsonden, 45 Minuten Stimulation mit 110 µA. 90 Minuten danach deutliche Schmerzreduktion. Am nächsten Tag Belastung des Beines möglich bei 80% Schmerzminderung. Nach 4 Tagen ohne weitere Therapie beschwerdefrei, Patient spielt wieder Fußball.

### Beispiel 14: Trigeminusneuralgie, mit untypischem, dauerhaftem Gesichtsschmerz rechts

Männlicher Patient, 67 Jahre. Trigeminusneuralgie seit 8 Jahren. Beginn nach Zahnbehandlung. Zustand nach Operation nach Janetta, Schmerzstärke unter täglicher Pregabalin-Einnahme von 8-9 auf der 11 point box scale. Zentrierung des Schmerzes auf die rechte Oberlippe. Erfindungsgemäße Therapie: Rechte Oberlippe 8 Nadeln 0,2 x 25 mm, kreisförmige Verkopplung, Anlegen der Kathode. Anode an Klebepad rechter Oberarm. Stimulation 30 Minuten 160 µA. 30 Minuten nach Therapie Reduktion des Dauerschmerzes an der rechten Oberlippe auf 3 (11 point box scale). Anhaltende Besserung für 2 Tage. Danach wieder ursprüngliche Schmerzintensität. Patient wiederholt die Behandlung 1-2 mal pro Woche. Jeweils Schmerzreduktion für ca. 2 Tage.

### Beispiel 15: Tinnitus

Weiblicher Patient, 73 Jahre, seit 12 Monaten Tinnitus re. Aufkleben einer 1 cm² großen Klebeelektrode 1 Querfinger unter dem Mastoid rechts, Aufkleben einer 50 cm² großen Klebelektrode über dem Deltoid rechter Oberarm. Verbindung der kleinen Elektrode mit der Anode, Verbinden der großen Elektrode mit der Kathode. Anlegen von 1000 µA über 45 Minuten. Minderung des Tinnitus um 30% direkt nach der Therapie, vollständiges Sistieren des Tinnitus einen Tag nach Therapie, erneuter Tinnitus mit gleicher Lautstärke wie vor der der Therapie am zweiten Tag nach Therapie. Replikation des Reaktionsmusters an drei aufeinander folgenden Therapien im Abstand von jeweils 2 Wochen.

### Beispiel 16: Tennisarm

47-jähriger Patient, seit 4 Monaten Tennisarm rechts. Aufkleben einer 1,5 cm² großen Klebeelektrode auf dem rechten Epikondylus lateralis, Aufkleben einer 50 cm² großen Klebelektrode über dem Deltoid rechter Oberarm. Verbindung der kleinen Elektrode mit der Anode, Verbinden der großen Elektrode mit der Kathode. Anlegen von 1500 µA über 60 Minuten. 4-malige Wiederholung der Therapie in 2-wöchigem Abstand. Besserung der Ellbogenschmerzes nach der ersten Therapie um 20%, nach der zweiten Therapie um 40%, nach der dritten Therapie um 60%, nach der vierten Therapie um 80%.

### Beispiel 17: Migräneartiger Kopfschmerz

51-jähriger Patient mit klopfendem Kopfschmerz li, occipital bis Schläfe ausstrahlend, Übelkeit, Erbrechen. Setzen einer ersten Nadel über dem Nervus occipitalis major und einer zweiten Nadel auf der Stirn, zwei Querfinger über dem Auge. Nadelstärke 0,25 mm. Anlagen der Kathode an die Stirnnadel und der Anode an die Occipitalnadel. Stromquelle 3 V. Batterie, Stromstärke 120 µA, im Verlauf der Behandlung auf 20 µA sich reduzierend. 80 % Besserung des Kopfschmerzes nach 12 Stunden.

### Beispiel 18: Spannungskopfschmerz

51-jähriger Patient mit dumpfem Kopfschmerz beidseits, occipital bis Stirn ausstrahlend. Setzen von vier Nadeln occipital an druckschmerzempfindlichen Bereichen an der Hinterhauptsschuppe und einem Klebepad ca 100 cm² auf der rechten Flanke unterhalb des Rippenbogens, Nadelstärke 0,3 mm. Anlegen der Kathode an die flächige Elektrode und der Anode an die Occipitalnadeln. Stromquelle 3 V. Batterie, Stromstärke 200 µA, im Verlauf der Behandlung auf 110 µA sich reduzierend. 50% Besserung des Kopfschmerzes nach 6 Stunden, am nächsten Tag kopfschmerzfrei.

### Beispiel 19: Entzündung der Supraspinatussehne re Schulter

Männlicher Patient, 48 Jahre, Wurfsportler, seit 5 Wochen Schulterschmerz re. Aufkleben einer 2 cm² großen Klebeelektrode über dem Tuberkulum majus der Schulter, Aufkleben einer 100 cm² großen Klebelektrode über der rechte Flanke, direkt unterhalb des Rippenbogens. Verbindung der kleinen Elektrode mit der Kathode, Verbinden der großen Elektrode mit der Anode. Anlegen von 1500 µA über 60 Minuten. Besserung des Schulterschmerzes um 20% direkt nach der Therapie, 70% Besserung am nächsten Tag.

### Beispiel 20: Herpes Zoster Neuralgie

Weiblicher Patient, 62 Jahre, seit 4 Wochen Herpes Zoster Neuralgie linke Flanke ca. BWK 6-9. Setzen von 15 0,3 mm starken Nadeln tangential unter die Haut direkt im schmerzhaften Areal. Aufkleben einer 50 cm² großen Klebelektrode über dem Deltoid rechter Oberarm. Verbindung der Nadeln einzeln mit der Kathode, Verbinden der Klebelektrode mit der Anode. Anlegen von 500 µA über 5 Minuten, danach 400 µA über 5 Minuten, danach 50 µA über 30 Minuten, Besserung des Neuralgieschmerzes am ersten Tag nach der Therapie um 50%, Nach einer Woche gleiche Behandlung, weitere Besserung um 50%. 4 Wochen später vollständige Schmerzfreiheit.

## Patentansprüche

1. Gleichstromabgabevorrichtung umfassend eine Gleichstromquelle oder eine Einrichtung zum Anschließen an eine Gleichstromquelle und eine erste Elektrode sowie eine zweite Elektrode zum Verbinden mit der Gleichstromquelle, wobei die Gleichstromabgabevorrichtung entweder ein Mittel zum Konstanthalten der Stromstärke bei der Abgabe des Gleichstroms umfasst oder die Gleichstromquelle eine Batterie ist, **dadurch gekennzeichnet, dass** die erste Elektrode als Mehrzahl miteinander elektrisch leitend verbundener Nadeln ausgebildet ist und 3-12 Nadeln umfasst und die zweite Elektrode entweder (i) als flächige Elektrode oder (ii) als Nadel oder Mehrzahl miteinander elektrisch leitend verbundener Nadeln ausgebildet ist.

2. Gleichstromabgabevorrichtung nach Anspruch 1, wobei die zweite Elektrode als flächige Elektrode ausgebildet ist.

3. Gleichstromabgabevorrichtung nach einem der vorherigen Ansprüche, ferner umfassend ein Mittel zum Konstanthalten der Stromstärke bei der Abgabe des Gleichstroms.

4. Gleichstromabgabevorrichtung nach Anspruch 3, wobei das Konstanthalten ein automatisiertes Konstanthalten ist.

5. Gleichstromabgabevorrichtung nach einem der vorherigen Ansprüche, wobei die Spitzen der Mehrzahl von Nadeln entlang eines im Wesentlichen kreisförmigen oder elliptischen Umfangs angeordnet sind und wobei die elektrisch leitende Verbindung der Nadeln vorzugsweise entlang des Umfangs ausgebildet ist.

6. Gleichstromabgabevorrichtung nach einem der vorherigen Ansprüche, wobei die erste Elektrode als Kathode ausgebildet ist.

7. Gleichstromabgabevorrichtung nach einem der vorherigen Ansprüche zur Anwendung bei der Behandlung von Entzündungen und/oder Schmerzen, insbesondere der Muskeln, Nerven, Sehnen oder Knochen.

8. Gleichstromabgabevorrichtung nach einem vorherigen Ansprüche, wobei entweder (A) die zweite Elektrode als flächige Elektrode ausgebildet ist und vorzugsweise die maximale Stromstärke des Gleichstroms 2000 µA beträgt oder (B) die zweite Elektrode als Nadel ausgebildet ist und vorzugsweise die maximale Stromstärke des Gleichstroms 100 µA beträgt.

9. Verwendung einer Gleichstromabgabevorrichtung nach einem der Ansprüche 1 bis 6 zur kosmetischen Behandlung des menschlichen oder tierischen Körpers.

10. Kit zum Herstellen einer Gleichstromabgabevorrichtung, vorzugsweise wie in einem der Ansprüche 1 bis 6 beschrieben, umfassend: eine Mehrzahl von Nadeln zur Verwendung als erste Elektrode, die 3-12 Nadeln umfasst, eine flächige Elektrode, eine Nadel oder eine Mehrzahl von Nadeln zur Verwendung als zweite Elektrode, entweder ein Mittel zum Konstanthalten der Stromstärke bei der Abgabe von Gleichstrom durch eine Gleichstromquelle oder eine Gleichstromquelle, die eine Batterie ist, und optional Mittel zum elektrisch leitenden Verbinden einer Mehrzahl von Nadeln.

11. Verfahren zum Herstellen einer Gleichstromabgabevorrichtung, vorzugsweise wie in einem der Ansprüche 1 bis 6 beschrieben, umfassend die folgenden Schritte: Bereitstellen eines Kits nach Anspruch 10, Bereitstellen einer/der Gleichstromquelle, elektrisch leitendes Verbinden der Mehrzahl von Nadeln zum Bilden der ersten Elektrode, Bereitstellen der flächigen Elektrode oder der Nadel oder elektrisch leitendes Verbinden der Mehrzahl von Nadeln zum Bilden der zweiten Elektrode, Verbinden der ersten Elektrode mit der Gleichstromquelle und Verbinden der zweiten Elektrode mit der Gleichstromquelle.

## Claims

1. Direct current output apparatus comprising a direct current source or a device for connecting to a direct current source and a first electrode and a second electrode for connecting to the direct current source, wherein the direct current output apparatus comprises either a means for keeping the current intensity constant when delivering the direct current or the direct current source is a battery, **characterised in that** the first electrode is formed as a plurality of needles which are electrically conductively connected to one another and comprises 3-12 needles and the second electrode is formed either (i) as a flat electrode or (ii) as needles or a plurality of needles electrically conductively connected to one another.

2. Direct current output apparatus according to claim 1, wherein the second electrode is formed as a flat electrode.

3. Direct current output apparatus according to any one of the preceding claims, further comprising a means for keeping the current intensity constant when delivering the direct current.

4. Direct current output apparatus according to claim 3, wherein keeping the current intensity constant is performed automatically.

5. Direct current output apparatus according to any one of the preceding claims, wherein the tips of the plurality of needles are arranged along a perimeter, which is essentially circular or elliptical, and wherein the electrically conducting connection of the needles is formed preferably along the perimeter.

6. Direct current output apparatus according to any one of the preceding claims, wherein the first electrode is formed as a cathode.

7. Direct current output apparatus according to any one of the preceding claims for use in the treatment of inflammations and/or pain, in particular in the muscles, nerves, tendons or bones.

8. Direct current output apparatus according to any one of the preceding claims, wherein either (A) the second electrode is formed as a flat electrode and preferably the maximum current strength of the direct current is 2000 µA or (B) the second electrode is formed as needles and preferably the maximum current strength of the direct current is 100 µA.

9. Use of a direct current output apparatus according to any one of claims 1 to 6 for the cosmetic treatment of the human or animal body.

10. Kit to produce a direct current output apparatus, preferably as described in any one of claims 1 to 6, comprising: a plurality of needles for use as the first electrode comprising 3-12 needles, a flat electrode, a needle or a plurality of needles to use as the second electrode, either a means for keeping the current intensity constant when delivering the direct current by means of a direct current source or a direct current source which is a battery, and optionally a means to connect a plurality of needles in an electrically conducting manner.

11. Method for producing a direct current output apparatus, preferably as described in any one of claims 1 to 6, comprising the following steps: production of a kit according to claim 10, producing a/the direct current source, connecting, in an electrically conducting manner, the plurality of needles to form the first electrode, producing the flat electrode or the needle or connecting, in an electrically conducting manner, the plurality of needles to form the second electrode, connecting the first electrode to the direct current source and connecting the second electrode to the direct current source.

## Revendications

1. Dispositif de délivrance d'un courant continu comprenant une source d'alimentation en courant continu ou un dispositif pour le branchement à une source d'alimentation en courant continu et une première électrode ainsi qu'une deuxième électrode pour la connexion à la source d'alimentation en courant continu, le dispositif de délivrance d'un courant continu soit comprenant un outil pour maintenir constante l'intensité du courant lors de l'émission du courant continu, soit la source d'alimentation en courant continu étant une batterie, **caractérisé en ce que** la première électrode est formée comme une multitude d'aiguilles reliées les unes aux autres de façon électro-conductrice et comprend 3-12 aiguilles et la deuxième électrode est formée soit (i) comme une électrode plane, soit (ii) comme une aiguille ou une multitude d'aiguilles reliées les unes aux autres de façon électro-conductrice.

2. Dispositif de délivrance d'un courant continu selon la revendication 1, la deuxième électrode étant formée comme une électrode plane.

3. Dispositif de délivrance d'un courant continu selon l'une quelconque des revendications précédentes, comprenant également un outil pour maintenir constante l'intensité du courant lors de l'émission du courant continu.

4. Dispositif de délivrance d'un courant continu selon la revendication 3, le maintien constant étant un maintien constant automatisé.

5. Dispositif de délivrance d'un courant continu selon l'une quelconque des revendications précédentes, les pointes de la multitude d'aiguilles étant disposées le long d'une circonférence essentiellement circulaire ou elliptique et la connexion électro-conductrice des aiguilles étant constituée de préférence le long de la circonférence.

6. Dispositif de délivrance d'un courant continu selon l'une quelconque des revendications précédentes, la première électrode étant formée comme une cathode.

7. Dispositif de délivrance d'un courant continu selon l'une quelconque des revendications précédentes destiné au traitement d'inflammations et/ou de douleurs, notamment des muscles, nerfs, tendons ou os.

8. Dispositif de délivrance d'un courant continu selon l'une quelconque des revendications précédentes, soit (A) la deuxième électrode étant formée comme une électrode plane et de préférence l'intensité du courant continu maximum étant de 2000 µA, soit (B) la deuxième électrode étant formée comme une aiguille et de préférence l'intensité du courant continu maximum étant de 100 µA.

9. Utilisation d'un dispositif de délivrance de courant continu selon l'une des revendications 1 à 6 pour le traitement cosmétique du corps humain ou animal.

10. Kit de fabrication d'un dispositif de délivrance de courant continu, de préférence tel que décrit dans l'une quelconque des revendications 1 à 6, comprenant : une multitude d'aiguilles pour être utilisées comme première électrode, qui comprend 3-12 aiguilles, une électrode plane, une aiguille ou une multitude d'aiguilles pour être utilisées comme deuxième électrode, soit un outil pour maintenir constante l'intensité du courant lors de l'émission du courant continu par une source d'alimentation en courant continu, soit une source d'alimentation en courant continu, qui est une batterie, et un outil en option pour la connexion électro-conductrice d'une multitude d'aiguilles.

11. Procédé de fabrication d'un dispositif de délivrance de courant continu, de préférence tel que décrit dans l'une quelconque des revendications 1 à 6, comprenant les étapes suivantes : mise à disposition d'un kit selon la revendication 10, mise à disposition d'une/de la source de courant continu, connexion électro-conductrice de la multitude d'aiguilles pour former la première électrode, mise à disposition de l'électrode plane ou de l'aiguille ou connexion électro-conductrice de la multitude d'aiguilles pour former la deuxième électrode, connexion de la première électrode à la source de courant continu et connexion de la deuxième électrode à la source de courant continu.
